# EUROPEAN PATENT SPECIFICATION

(11) **EP 1 545 563 B1**
(45) Date of publication and mention of the grant of the patent: **16.04.2008**
(21) Application number: 03795536.6
(22) Date of filing: 12.09.2003
(51) Int. Cl.: A61K 31/718, A61K 9/16, A61P 1/10, A61P 41/00

(54) **COMPOSITION AND METHOD FOR USE IN INTESTINAL CLEANSING PROCEDURES**
ZUSAMMENSETZUNG UND VERFAHREN ZUR VERWENDUNG BEI DARMREINIGUNGSVERFAHREN
COMPOSITION ET PROCEDE A UTILISER DANS DES PROCEDURES DE NETTOYAGE INTESTINAL

(30) Priority: 13.09.2002 SE 0202723; 12.11.2002 US 425320 P
(43) Date of publication of application: 29.06.2005
(73) Proprietor: Metcon Medicin AB, 181 70 Lidingö (SE)
(72) Inventor: LAKE, Mats, S-181 41 Lidingö (SE); SMITH, Ulf, S-540 33 Fjärås (SE); AXELSEN, Mette, S-421 65 V Frölunda (SE); OLAUSSON, Eva, S-431 32 Mölndal (SE)
(74) Representative: Holmberg, Martin Tor
(86) International application number: PCT/SE2003/001428
(87) International publication number: WO 2004/024168

(56) References cited:
- WO-A1-95/24906
- CA-A1- 2 007 387
- DE-A1- 3 705 874
- GB-A- 1 414 121
- US-A- 5 624 907
- US-A- 5 997 906
- US-A- 6 162 464
- US-B1- 6 316 427

## Description

The present invention concerns the field of intestinal cleansing, irrespective of the intestinal cleansing being performed as a preparatory step before examination of the intestines, intestinal surgery, or for other purposes. The invention relates to a composition and a method of its use.

### Background of the invention

Proper intestinal cleansing is crucial for successful examination or treatment in certain medical procedures. For example, in patients undergoing colonoscopy, colon x-ray or intestinal surgery, it is important that the colon or the intestines is/are completely emptied with regard to faecal matter prior to the examination/operation in order to obtain satisfactory results. The elimination of gas is also important, in particular when polypectomy is performed, as intestinal gases mixed with faecal material can ignite during electrocautery.

Inadequate intestinal cleansing has many negative consequences, either directly or indirectly:
- remaining faecal matter can prevent the discovery of malignant tumours/polyps
- if perforation occurs, residual faeces could cause peritoneal contamination
- an unsuccessful procedure causes patient discomfort, with no benefit
- valuable time is lost for both the patient and the physician
- the patient needs to be rescheduled for a new examination
- patient anxiety is prolonged with the wait for a second procedure
- the intestinal cleansing requires time and adherence to dietary restrictions, and may be more difficult to complete a second time.

In addition to the discomfort experienced by the patient during the actual examination of the colon or intestines, the intestinal cleansing procedure itself, most often, is an unpleasant experience for the patient. Recorded side effects include hunger, nausea, vomiting, headache, tiredness, mood swings, irritation, concentration difficulties, allergic reactions and abdominal cramps. For elderly patients or patients with, for example, diabetes or other nutritional disorders that require special diets, the side effects may be more serious or even dangerous. Thus, there is a need for an adjunct therapy and/or alternative approaches to intestinal cleansing compositions and methods that address the above side effects.

In particular, a composition, which reduces or eliminates the nutritional deficiency, such as hypoglycaemia, often experienced during the period of fasting and cleansing, would be beneficial. In this context, hypoglycaemia is defined as mild (a blood glucose level about 2.4-4.0 mmol/l) and severe (a blood glucose level below 2.4 mmol/l). It is important that this condition is avoided, regardless of the general state of health of the patient. Naturally, compositions which are applicable to all patients, are desired, i.e. composition adapted to the special needs of weakened, elderly or diabetic patients.

### Prior art

Over the years, many different laxatives and intestinal cleansing compositions have been developed. Some of them are still in use and others are not. Desirable criteria are that the composition should be safe, simple and acceptable to patients. It should also be reliable with respect to emptying the colon of all faecal matter, most liquid material, and at the same time it should not affect the appearance of the colon. Furthermore, it should be easy for the patient to self-administer over a short period of time, have a pleasant taste, and exert activity rapidly following administration. Commonly used laxative products include castor oil, magnesium citrate, bisacodyl, polyethylene glycol (PEG) in combination with electrolytes and sodium phosphate.

The use of sodium phosphate, a saline laxative, has become widespread. It acts by osmotically retaining water in the intestines causing watery diarrhoea. There are however problems associated with the ingestion of sodium phosphate, such as fluid and electrolyte imbalances and acidosis. Sodium phosphate may also cause changes to the colonic mucosa that mimic inflammatory bowel disease (IBD), rendering accurate diagnosis more difficult.

Polyethylene glycol (PEG) solutions in combination with electrolytes are also frequently used for intestinal cleansing. This treatment often gives very adequate levels of intestinal cleansing with minimal fluid and electrolyte shift. However, also this treatment has major drawbacks for the patient who, for example, still needs to drink large volumes of the solution with the consequence of volumogenic diarrhoea.

Thus, both the use of phosphate solutions and PEG can achieve adequate and comparable intestinal cleansing but are still suffering from unpleasant taste, and excessive volume as well as other side-effects. Since there is a requirement on solution isotonicity, the addition of water-soluble adjuvants to alter, for example, the taste or to minimise the troublesome side-effects, may not always be possible. The addition of other substances that could be fermented by the intestinal flora should also be avoided since the production of gas may constitute an explosion hazard especially in the case of colonoscopy with electrocautery. However, according to prevailing clinical praxis, the patients are often recommended to ingest sucrose to prevent severe drops of the blood glucose level. For long-term blood glucose management, and in particular for diabetic patients, this does not constitute a good alternative.

The use of complex carbohydrates and in particular native cornstarch for blood glucose management in diabetic patients has been disclosed, e.g. in WO 02/34271 where also a cornstarch composition exhibiting controlled enzymatic degradation is presented. The beneficial value of complex carbohydrates is also disclosed in U.S. 6,316,427, which describes a method for improving glucose tolerance in diabetic patents, said method comprising ingesting a therapeutic amount of native cornstarch at bedtime.

Nutritional supplements comprising, among other ingredients, various amounts of complex carbohydrates, have been developed. U.S. 5,968,896 discloses a nutritional supplement comprising approximately from about 10 to about 75 grams carbohydrate; approximately from about 5 to about 50 grams protein; approximately from about 3 to about 30 grams fat; and a therapeutic amount of antioxidant, for use in weight maintenance in individuals scheduled to undergo major surgery to prevent or reduce postoperative complications.

While mentioning native cornstarch, the '896 patent gives an abundant list of other ingredients, many of which as such could interfere with the visual inspection of the intestines (e.g. colouring agents, fibres) or induce the production of faecal matter interfering with either the inspection or with surgical intervention (proteins, fats, simple carbohydrates).

U.S. 5,624,907 on the other hand describes a beverage for preoperative use, comprising a hypotonic aqueous solution of a carbohydrate mixture consisting of at least one member from each of the groups, mono-, di-, and polysaccharides in an amount of 8-20, preferably 9-15 grams carbohydrates/100 ml solution. The '907 patent also relates to a hypotonic aqueous solution as defined above for the use as a beverage for preoperative intake, to the use of mono-, di- and polysaccharides for the preparation of such a hypotonic aqueous solution and to the use of a dry substance comprising essentially A) a carbohydrate mixture consisting of 10-30% by weight of at least one monosaccharide, 10-30% by weight of at least one disaccharide and at least one polysaccharide as the balance to 100% by weight of said mixture and B) minor amounts of salts, flavouring and preservatives for the preparation of a hypotonic beverage for preoperative intake. Finally there is also disclosed a method for suppressing the negative influence on patient carbohydrate metabolism after surgery, and improving the defence capacity of the patient upon bleeding in connection with or after the operation, which method comprises preoperative oral administration to the patient an effective amount of a hypotonic aqueous solution as defined above.

As the '907 patent focuses on administering a maximum of carbohydrates, there is a reason to believe that the effects on blood glucose would be considerable and bring about the need for insulin intervention in diabetic patients.

The aim of the present invention is to make available a product that improves the control and the stabilisation of the blood glucose level during intestinal cleansing, which gives a more agreeable and safe preparation, particularly with regard to patients with already precarious health. It may not compromise the effect of the laxative or negatively influence the cleanness of the intestines, as well as having a pleasant taste and improve the well being of the patient

Further problems encountered in the relevant field, and the corresponding solutions and advantages offered by the invention, will be evident from the following description and examples.

### Summary of the invention

The present inventors have shown that a complex carbohydrate, either alone or in combination with one or more rapidly digested carbohydrates, can be used to reduce or eliminate the nutritional deficiency, in particular hypoglycaemia, often experienced during the period of fasting and cleansing. The composition according to the invention has also been shown to improve the subj ective well being of the patient, an important factor supporting compliance with the prescribed treatment. The invention makes available a new use of native corn starch, and composition as defined in the attached claims, hereby incorporated by reference.

### Short description of the drawings

The invention will be described in closer detail in the following description, non-limiting examples, and attached claims, with reference to the drawings, in which
Fig. 1A shows the variations in blood glucose (mmol/l) measured at 30 minute intervals, where three groups of test subjects were given either a normal meal tailored for diabetic patients (•), a composition according to the invention (○), or treated according to prevailing practice, i.e. given sucrose-containing clear drinks (Δ);
Fig. 1B shows the variations in blood glucose (mmol/l) in four patients, using a composition according to one embodiment of the invention;
Fig. 2A shows bowel cleanness score for different bowel sections, obtained in a 69 patient study, where prevailing practice and the inventive concept were compared with respect to cleanness in different intestinal sections. No significant difference in the effectiveness was seen;
Fig. 2B shows the same comparison as in Fig. 2A, with the exception that only the results of patients with excellent compliance were included (n = 52);
Fig. 3A illustrates the subjective symptoms experienced in the same 69 patient study, showing that patients following the inventive treatment experienced significantly less adverse symptoms; and
Fig. 3B shows the same comparison as in Fig. 3A, with the exception that only the results from patients with excellent compliance were included (n = 52).

### Description

Pure starch, although theoretically a good source of glucose and free from surplus calories, is practically impossible to ingest. The starch powder itself lacks taste and feels extremely dry and sandy in the mouth. A suspension, e.g. in water, tends to sediment quickly, and has a disagreeable texture.

Different sources of starch include, but are not limited to, grains, root-crops (mainly potato), and leguminous plants. According to the present invention, cornstarch is preferred.

Cornstarch consists of granules sized 2 = 32 µm, mainly comprising two components, amylose and amylopectin. Amylose has a linear structure while amylopectin is branched. Both amylose and amylopectin consist of α-(1,4)-linked glucose residues while amylopectin also has α-(1,6)-linked glucose residues. The starch granules are insoluble in cold water and swell in warm. The swelling is reversible until the temperature reaches about 55 to 65°C. At this temperature the starch granules gelatinise and loose their crystalline structure.

The degradation of starch is catalysed by α-amylase, which in humans is present in the saliva and in the small intestine. The digestibility of starch, both *in vivo* and *in vitro* depends on the source of starch as well as its pre-treatment (e.g. native, fine / coarse, gelatinised or chemically modified). In the present description, claims and examples, the term "native starch" is used to define starch that has not been subjected to heat-treatment or chemical treatment. The term "native starch" thus comprises the vegetable and/or plant seeds, kernels or grains, as well as mechanically treated fractions, such as the milled and sieved product, granules and flour.

The present inventors have found that a complex carbohydrate can be used to reduce or eliminate the nutritional deficiency, in particular hypoglycaemia, often experienced during the period of fasting and cleansing. Surprisingly, this is possible without any negative influences on the result of the intestinal cleansing, as the complex carbohydrate is enzymatically degraded in the small intestine, and the degradation products efficiently absorbed.

The composition of the present invention is preferably used together with or co-administered with conventional preparations for intestinal cleansing. In this context, the terms co-administration and adjunct therapy will be used, their meaning including simultaneous, substantially simultaneous, sequential and intermediate administration of the inventive composition before or preferably during intestinal cleansing.

In a first aspect of the invention, the present invention thus makes available the use of a complex carbohydrate for the manufacture of a composition, e.g. a pharmaceutical formulation for intestinal cleansing, wherein said complex carbohydrate is present in an amount and form, ingestion of which provides a blood glucose level of at least 3.5 mmol/l, preferably about 4 mmol/l, during the course of the intestinal cleansing. Most preferably, ingestion of the composition will result in a blood glucose response similar to that corresponding to a normal meal in healthy persons, or in diabetics taking their normal medication. Most preferably the complex carbohydrate is native cornstarch.

According to a preferred embodiment of the invention, the dose for a person weighing 75 kg and having an estimated energy requirement of 2400 kcal/day was calculated to supply a total of 360 g carbohydrates. This is estimated to cover about 60 % of the daily energy requirement, and probably approximately 100 % of the carbohydrate requirement. Further, this dose of 360 g was subdivided into 3 meals and 3 snacks to suit insulin treated patients, i.e. breakfast, snack, lunch, snack, dinner, snack, and evening meal. The corresponding carbohydrate doses were 47g, 28 g, 112 g, 28 g, 112 g, 28 g, and 47 g respectively.

In the context of the present invention, the term intestinal cleansing encompasses all procedures for intestinal cleansing, regardless if it is performed as a step in the pre-treatment of patients scheduled to undergo colonoscopy, sigmoidoscopy, other investigation of the intestines, such as a barium enema investigation of the intestines, colon or intestinal x-ray, or intestinal surgery.

In a second aspect of the invention, the present invention makes available a composition, e.g. a pharmaceutical formulation wherein said composition contains a complex carbohydrate in a form suitable for oral ingestion, said complex carbohydrate being present in an amount and form, ingestion of which provides a blood glucose level of at least 3.5 mmol/l, preferably about 4 mmol/l, when taken at certain intervals, for instance resembling a normal habitual meal pattern. Most preferably, ingestion of the composition will result in a blood glucose response similar to that corresponding to a normal meal in healthy persons, or in diabetics taking their normal medication. Preferably said complex carbohydrate is native cornstarch.

According to one embodiment of the invention, the complex carbohydrate is accompanied by a more rapidly digestable carbohydrate, such as sucrose or dextrose. According to a preferred embodiment, the complex carbohydrate is partially pre-gelatinised, preferably by pre-gelatinising the surface layer of the carbohydrate particles or granules. In addition to providing small amounts of a rapidly digestable carbohydrate, the pre-gelatinisation influences the sedimentation properties and helps to prevent the rapid sedimentation typical for many carbohydrate suspensions.

In the above mentioned daily dose of 360 g carbohydrates, it is preferred that a simple carbohydrate, e.g. sucrose, accompanies the complex carbohydrate, preferably uncooked cornstarch in the proportion of at least 1:5, preferably 1:4 and most preferably 1:3. Consequently, in the above doses, the 47 g breakfast and evening meal contains 12 g sucrose and 35 g uncooked cornstarch, the 28 g snack correspondingly 7 g and 21 g, and the lunch and dinner correspondingly 29 g and 83 g.

This combination of uncooked starch and a simple carbohydrate is important for diabetic patients, and it is contemplated that a composition intended for healthy patients would consist of 100 % uncooked cornstarch, since this could further smoothen the blood glucose fluctuations. On the other hand, the rapid-acting carbohydrates, e.g. sucrose, may affect satiety in a favourable manner.

According to an embodiment of the invention, said composition is in the form of a powder for mixing in an aqueous or non-aqueous liquid. According to another embodiment, said composition is in the form of a tablet or capsule, suitable for oral ingestion.

According to yet another embodiment, said composition is in the form of an effervescent tablet suitable for dissolving in an aqueous or non-aqueous liquid. Preferably, in both the above embodiments, said complex carbohydrate is present in a form, which prevents or delays sedimentation when the composition is mixed in an aqueous solution. This is achieved i.a. by pre-gelatinisation or by granulating the starch micro granules with a substance, resulting in aggregated granules being at least partially encapsulated in the substance.

Suitable substances are non-toxic substances, suitable for ingestion, such as substances generally recognised as safe (GRAS) and approved for use in pharmaceutical applications and/or in food products. A non-exclusive list of suitable substances includes polymers such as gum arabicum, potassium alginate, guar gum, methyl cellulose, ethyl cellulose; liquid oils, liquid and hard fats and waxes, such as paraffin, hydrogenated cottonseed oil, beeswax, and carnauba wax. According to one embodiment, presently preferred by the inventors, said native cornstarch is coated with a layer of pre-gelatinised starch.

According to a further embodiment of the invention, the composition further includes flavouring agents and colouring agents that do not interfere with the inspection of the intestines and/or surgical intervention. According to one embodiment, the composition also contains sodium bicarbonate to create effervescence and/or to neutralize or buffer stomach acids.

In the above, the term "an aqueous or non-aqueous liquid" is meant to encompass both water as such, water including additives with regard to taste and consistency, as well as solutions for intestinal cleansing purposes, such as electrolyte solutions or PEG solutions.

By reducing or eliminating the nutritional deficiency, in particular hypoglycaemia, often experienced during the period of fasting and cleansing, the present invention offers many advantages. One is that the patient's general state of health before, during and after the intestinal examination or surgery is improved. This will also result in better compliancy and faster recovery. Importantly, the health risks for particular patient groups, such as diabetics and elderly, are significantly reduced.

It is surprising that a complex carbohydrate could be used in this context, as it would be expected that a complex carbohydrate would contaminate the intestines because of bacterial degradation of the dietary fibre and resistant starch, leading to the production of faeces.

Further embodiments and advantages of the invention will be evident to a skilled person in light of the description, non-limiting examples and claims.

### Examples

### Example 1. Blood glucose levels

### Design of the study

Patients with Type-I insulin dependent diabetes participated in the investigation. Two investigations were performed, one main study and one sub-study. The characteristics of the patients can be seen in Table 1.

**Table 1. Characteristics of the patients**

| | Main study | Sub-study |
|---|---|---|
| | Average±SD | Average±SD |
| Age (year) | 55±10 | 46±17 |
| Male/female (n) | 6/6 | 2/2 |
| BMI (kg/length²) | 25±3 | 26±1 |
| HbA1c (%) | 6.3±0.7 | 6.3±0.7 |
| Insulin regimen (A/H)* | 6/6 | 0/4 |
| Diabetes duration (year) | 25±9 | 21±11 |

| | | |
|---|---|---|
| *A = Actrapid^{®} (a rapid acting, short duration human insulin from Novo Nordisk Scandinavia AB, Malmö, Sweden); H = Humalog^{®} (a rapid acting, short duration human insulin analogue from Eli Lilly Sweden AB, Stockholm, Sweden) | | |

Three meals with similar carbohydrate content were given in randomized cross-over design:
1. Treatment according to the prevailing practice, e.g. soft drinks containing table sugar for lunch and snack, and a reduction in the insulin dose by 50%.
2. Diabetic meal, e.g. a rice pilaf for lunch and a pumpernickel sandwich and an orange for snack, with maintenance of the regular insulin dose.
3. Solution B, e.g. table sugar and cornstarch in the proportions 1:3 for lunch and snack (for details, see Table 2). Also this regimen maintained the regular insulin dose.

**Table 2. Composition of solution B**

| | Lunch | Snack |
|---|---|---|
| Table sugar | 29 g | 7 g |
| Cornstarch | 83 g | 21 g |
| FUN Light™ concentrate* | 30 g | 15 g |
| Water | 370 g | 170 g |

| | | |
|---|---|---|
| * Non-caloric concentrated soft drink, added for palatability. | | |

The main study included 12 patients. Blood samples were taken was sampled for 5 hours, before and every half hour after consumption of the lunch and following the snack. The snack was given 150 minutes after the lunch. At meals 2 and 3, the patients used their regular dose of insulin prior to the meal. However, according to the prevailing clinical practice, the patients reduced their insulin dose to half during test meal 1. In case of a drop in blood glucose levels below 3.5 mmol/l, tablets containing dextrose were given until the blood glucose level returned to over 3.5 mmol/l.

In the sub-study an additional 4 patients were included. These four patients treated their diabetes with Humalog^{®} in connection to the meals and were only given solution B. In contrast to the main study the snack was given after 120 minutes to investigate if the blood glucose level could be further stabilised by this design.

### Statistics

Friedman's analysis of variance and Wilcox's Signed Rank Test were used to calculate significances. Due to the large day-to-day variation in blood glucose levels in patients with diabetes type-1, p<10 was chosen as significance level.

### Results

Hypoglycaemia was observed after time 150 minutes in all three groups but with different frequency and to different extent in the different groups. The degree of hypoglycaemia was estimated by the amount of dextrose ingested to balance the insulin levels in the patients (Table 3).

**Table 3. Dextrose intake (No. of tablets)**

| | Diabetic meal (n) | Prevailing practice (n) | Solution B (n) |
|---|---|---|---|
| Number of patients with hypoglycaemia | 1 | 2 | 1 |
| Total number of dextrose tablets | 2 | 8+13 | 2 |

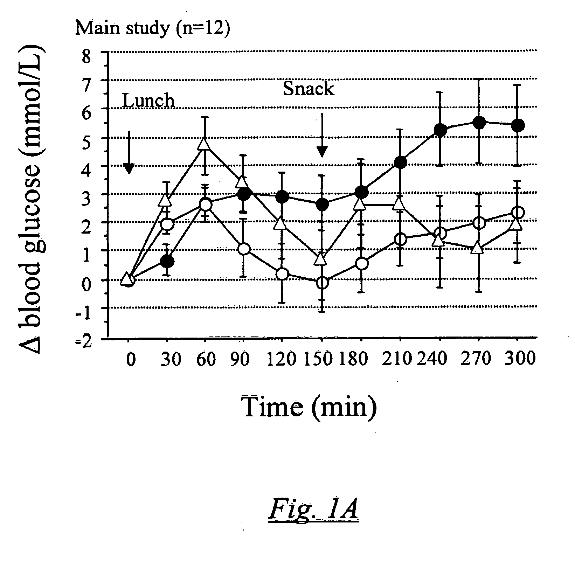

Main study (Figure 1A): Ingestion according to prevailing practice (group 1) resulted in a higher blood glucose response after the meal (5.1 ± 0.9 mmol/l) compared to group 2 (4.2 ± 0.7 mmol/l, p = 0.091) and group 3 (3.5 ± 0.6 mmol/l, p = 0.071). On the other hand there were no differences in the blood glucose levels at time 150 minutes (i.e. prior the snack) between group 1 and group 3, whereas group 2 had a higher level than group 3 (2.7 ± 0.9 vs. 0.0 ± 1.0 mmol/l, p = 0.060). After the snack, i.e. between 150 - 300 minutes, there were no significant differences in IAUC values between group 1 (262 ± 219 mmol/l * min, p =0.041) and group 3 (198 ± 137,p = 0.023), but the IAUC value for group 2 (657 ± 185 mmol/l*min) was higher than for the two other groups.

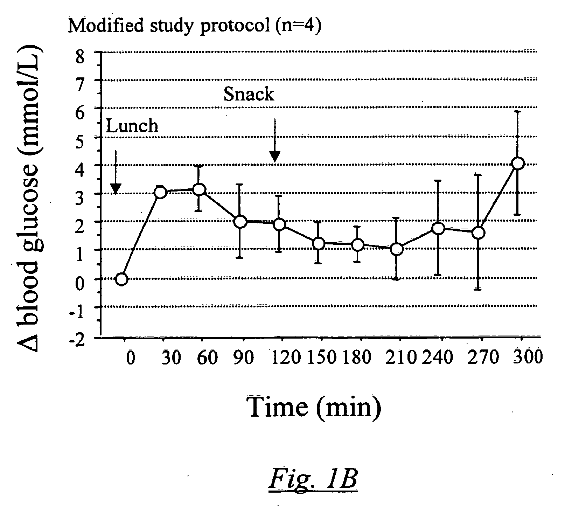

Sub-study (Figure 1B): By receiving the snack between meals (Solution B) 30 minutes earlier (compared to the main study) the blood glucose values were more stabilized, with a greater margin to the baseline value. The blood glucose increase was between 1 and 4 mmol/l postprandially, compared to between 0 and 3 in the main study. This shows that receiving the snack after 120 minutes further stabilises the blood glucose level. One patient experienced hypoglycaemia after receiving the snack, but the mean glucose level was similar whether excluding this individual or not.

### Discussion

Intake of Solution B led to a moderate average blood glucose increase that was kept at or over the baseline value during the entire postprandial period. Patients treated according to the prevailing practice could not balance the insulin effect and two patients had to eat between 8 and 13 dextrose tablets, respectively, despite the reduction of the dose of insulin in this group. This illustrates the problems associated with the prevailing clinical praxis. In contrast, only 2 dextrose tablets were needed after intake of solution B and the optimal diabetic meal respectively, demonstrating an equally good protection against hypoglycaemia with these two regimens. Furthermore, the results demonstrates that it is possible to use a full dose of insulin together with Solution B and still have a satisfying balance in the blood glucose level. Additional stabilisation was obtained with intake of Solution B when the snack between meals was ingested at 120 minutes after the meal, instead of 150 minutes. Thus, the use of Solution B stabilises the blood glucose level of the patient better (i.e. less hyper- and hypoglycaemia) than during treatment according to the prevailing practice and the patients can continue with their normal insulin treatment.

### Example 2. Bowel preparation

### Design of the study

Patients that have been referred to colonoscopy and to ingest the intestinal cleanser Phosphoral^{®} (an oral solution for intestinal cleansing, Ferring Läkemedel AB, Limhamn, Sweden) were included in the study. The patients were mainly non-diabetic, but also some patients with diabetes participated. The patients were randomly consecutively treated either according to prevailing practice or with Solution B. The day before colonoscopy the patients had their normal breakfast and thereafter adhered either to treatment according to the prevailing practice, i.e. sweetened soft drinks, or to ingestion of Solution B. The composition of solution B during the various test meals is shown in Table 4.

**Table 4. Composition of solution B**

| | Snack | Lunch | Snack | Dinner | Evening meal | Breakfast the second day |
|---|---|---|---|---|---|---|
| Table sugar | 7 g | 29 g | 7 g | 29 g | 12 g | 12 g |
| Cornstarch | 21 g | 83 g | 21 g | 83 g | 35 g | 35 g |
| FUN Light™ concentrate* | 11 g | 13,5 g | 11 g | 13,5 g | 11 g | 11 g |
| Water | 289 g | 370 g | 289 g | 370 g | 290 g | 290 g |

| | | | | | | |
|---|---|---|---|---|---|---|
| * Non-caloric concentrated soft drink, added for palatability. | | | | | | |

The patients were interviewed the day of the colonoscopy before the examination about subjective symptoms of the intestinal cleansing and compliance to the treatment. During the colonoscopy the gastroenterologist completed a questionnaire where the completeness of the bowel preparation was judged by the presence or absence of fecal residues in each of several segments of the colon. The questionnaire is based on a numerical evaluation where number 1 means that the colon is completely clean and number 7 means that it is completely blocked by faeces. To simplify the evaluation of the results the numbers were summarised and the results divided into three relevant categories: 1) Clean or liquid that can be removed 2) Faeces cover <25% of the surface 3) Faeces cover >25% of the surface. Each segment of the colon was evaluated separately (Table 5).

**Table 5. Degree of intestinal cleansing after intake of Solution B and the prevailing practice**

| (The numbers correspond to the number of patients in each category) | | | |
|---|---|---|---|
| **Solution B (n=5)** | | | |
| | Clean or liquid that can be removed | Faeces cover <25% of the surface | Faeces cover >25% of the surface |
| Rectum | 4 | 1 | 0 |
| Colon sigmoideum | 3 | 2 | 0 |
| Colon descendens and flexura hepatica | 3 | 2 | 0 |
| Colon transversum | 3 | 2 | 0 |
| Colon ascendens, caecum and flexura hepatica | 2 | 2 | 1 |
| Summary | 15 | 9 | 1 |
| **Prevailing practice (n=5)** | | | |
| | Clean or liquid that can be removed | Faeces cover <25% of the surface of | Faeces cover >25% the surface |
| Rectum | 4 | 1 | 0 |
| Colon sigmoideum | 4 | 1 | 0 |
| Colon descendens and flexura hepatica | 3 | 2 | 0 |
| Colon transversum | 3 | 2 | 0 |
| Colon ascendens, caecum and flexura hepatica | 2 | 3 | 0 |
| Summary | 16 | 9 | 0 |

### Results

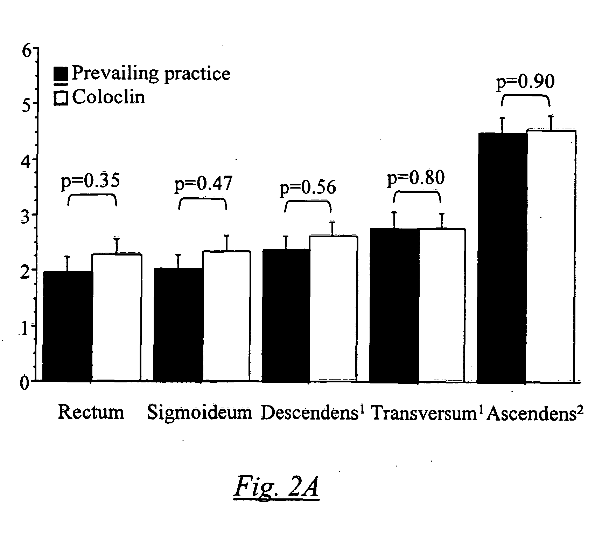

The results are shown in Fig. 2A showing mean values of cleanness for prevailing practice and using a composition according to the invention (Coloclin). The study comprised 69 patients. For the intestinal sections descendens and transversum, data was only obtained in 68 patients, and for ascendens, only in 63 patients.

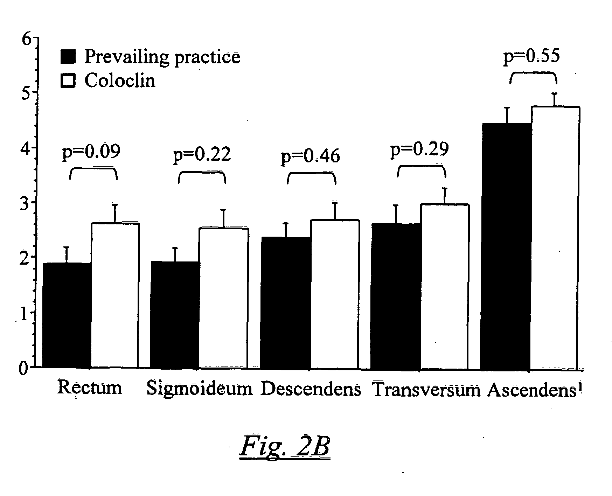

Fig. 2B shows the results the same study, wherein only patients with excellent complience were included (n=52). For ascendens, data was obtained only in 49 patients. The results indicate an improved efficacy in the favour of the composition according to the invention.

The intestinal cleansing is strikingly similar in all segments of the colon, which is also shown by the number of patients within each category. The only difference that can be observed is in the group that had Solution B where one patient could not be assigned the cleanest category in the evaluation of colon sigmoideum as well as colon ascendens, caecum and flexura hepatica. However, the preparation of those segments was sufficient to carry out the examination. The results suggest that Solution B gives equally good preparation as the prevailing clinical practice and that both of these means of cleaning the intestines gave generally satisfactory results. The results of Fig. 2B indicate that a composition according to the invention is superior to prevailing practice.

### Example 3. Clinical studies

Further studies have been performed after the priority date, but before the international filing dates. These studies, involving a considerable number of patients (n=69) and performed according to strict clinical practice confirmed the feasibility of the inventive concept and underlined the advantages of the composition and method in healthy and diabetic patients alike.

In one study, the effect of the inventive composition on the postprandial blood glucose balance in patients with insulin treated type 1 diabetes was investigated. The inventive composition and method was compared to on the one hand, the prevailing practice, that is the administration of sucrose-containing clear drinks and a reduction in the insulin dose to 50 %, and on the other hand, an ideal diabetic diet with maintained regular insulin dose.

It was shown that the inventive composition and method was successful in stabilizing blood glucose values, and that an improved margin to the base line value was achieved. The main study involved 12 patients, and in a modified study, where the snack between meals was administered 30 minutes earlier, 4 patients took part.

In another study, the inventive composition and method was tested together with the ingestion of the intestinal cleansing agent Phosphoral^{®}. Six patients participated. It was shown that the inventive composition and method was successful in balancing the blood glucose level, as compared to prevailing practise.

In a third study, 69 patients were investigated with respect to intestinal cleanness and subjective symptoms following prevailing practice (Phosphoral^{®} and sucrose-containing drinks) and the inventive composition and method together with Phosphoral^{®}. There were no significant differences in the effectiveness of intestinal cleansing between the two. However, the subjective symptoms reported by the patients using a Visual Analogue Scale (VAS) form exhibited significant differences. The reported symptoms such as hunger, physical fatigue, concentration difficulties, mood swings, headache and incapacity for work were significantly lower for the patients receiving the inventive composition. Symptoms such as nausea, bloatedness, flatulence and other stomach complaints were reported to be equal for the two treatment methods. This is probably due to the character of the intestinal cleansing procedure itself, where very large amounts of water must be ingested. In this respect, it is an additional advantage of the present invention that the composition and method can be administered with comparatively little added water, compared to the amounts of water ingested during the procedure.

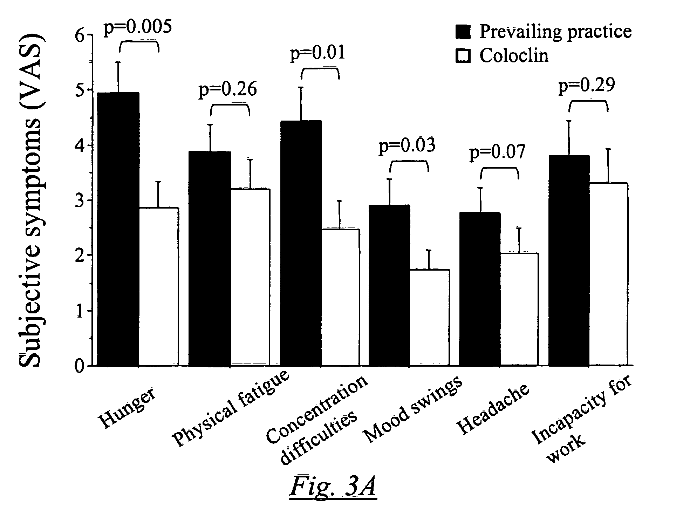

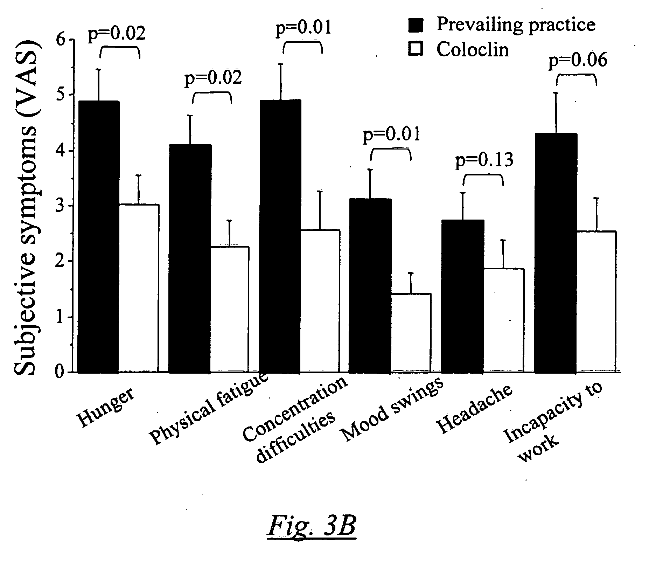

The subjective symptoms are presented in Fig. 3A and 3B, where the former represents the results obtained for all 69 patients included in the study, and the latter the results when only the patients with excellent compliance were included.

It has thus been shown that the inventive composition gives the same and most likely even better protection against hypoglycaemia in diabetic patients compared to the prevailing practice of sucrose-containing clear drinks despite the maintained normal insulin dose. In healthy patients, the inventive composition and method gives an improved blood glucose balance, and in all patients significantly reduced adverse effects, such as hunger, physical fatigue, concentration difficulties, mood swings, headache and incapacity for work. With regard to intestinal cleanness, the inventive method compares favourably to prevailing practice and does not interfere with the ability to perform colonoscopy and does not increase the faecal output. Importantly, the improved general well-being of the patients was seen as a considerable advantage of the inventive composition and method. Such benefits were observed equally in healthy and diabetic patients.

When using the inventive composition in clinical practice, it will be used as an adjunct therapy, combined with a laxative agent. In the studies performed by the present inventors, the laxative agent was Phosphoral^{®} (Ferring Läkemedel AB, Sweden) but it is understood that any other suitable laxative agent can be employed.

Although the invention has been described with regard to its preferred embodiments, which constitute the best mode presently known to the inventors, it should be understood that various changes and modifications as would be obvious to one having the ordinary skill in this art may be made without departing from the scope of the invention as set forth in the claims appended hereto.

## Claims

1. Use of native corn starch for the manufacture of a composition for co-administration_during procedures for intestinal cleansing, **characterized in that** said corn starch is present as granules in an amount and form, ingestion of which provides a blood glucose level similar to that following a normal meal in healthy persons, or in a diabetic person taking their normal medication, and leaves no faecal residue.

2. The use according to claim 1, wherein the blood glucose level is maintained at least 3.5 mmol/l, preferably at about 4 mmol/l, during the course of said intestinal cleansing.

3. The use according to claim 1, wherein the intestinal cleansing is a step in the pre-treatment of patients scheduled to undergo colonoscopy.

4. The use according to claim 1, wherein the intestinal cleansing is a step in the pre-treatment of patients scheduled to undergo sigmoidoscopy.

5. The use according to claim 1, wherein the intestinal cleansing is a step in the pre-treatment of patients scheduled to undergo barium enema investigation of the intestines.

6. The use according to claim 1, wherein the intestinal cleansing is a step in the pre-treatment of patients scheduled to undergo intestinal x-ray.

7. The use according to claim 1, wherein the intestinal cleansing is a step in the pre-treatment of patients scheduled to undergo intestinal surgery.

8. The use according to claim 1, wherein said composition further comprises a simple carbohydrate in the proportion of at least 1:5, preferably at least 1:4 and most preferably about 1:3 to the complex carbohydrate.

9. The use according to claim 8, wherein said simple carbohydrate is sucrose.

10. A composition for oral ingestion, said composition leaving essentially no faecal residue, **characterized in that** said composition contains
- native corn starch in a form suitable for such ingestion, said corn starch being present as aggregated granules, produced using a substance chosen among gum arabicum, potassium alginate, guar gum, methyl cellulose, ethyl cellulose, paraffin, hydrogenated cottonseed oil, beeswax, carnauba wax and pre-gelatinised starch, in an amount and form, ingestion of which provides a blood glucose level of at least 3.5 mmol/l, preferably about 4 mmol/l, when taken at intervals corresponding to a normal habitual meal pattern, and
- simple carbohydrates the ingestion of which gives a blood glucose level corresponding to the blood glucose level obtained after ingestion of a normal meal.

11. The composition according to claim 10, wherein said simple carbohydrate is sucrose.

12. The composition according to claim 10, wherein the simple carbohydrate or - hydrates and the native corn starch are present in a proportion of at least 1:5, preferably at least 1:4 and most preferably about 1:3.

13. The composition according to any one of claims 10 - 12, wherein said native cornstarch is coated with a layer of pre-gelatinised starch.

14. The composition according to any one of claims 10 - 12, wherein said composition is in the form of a powder for mixing in an aqueous or non-aqueous liquid.

15. The composition according to any one of claims 10 - 12, wherein said composition is in the form of an effervescent tablet suitable for dissolving in an aqueous or non-aqueous liquid.

16. The composition according to any one of claims 10 - 12, wherein said composition is in the form of a tablet or capsule suitable for oral ingestion.

## Patentansprüche

1. Verwendung von nativer Maisstärke zur Herstellung einer Zusammensetzung zur gleichzeitigen Verabreichung während Methoden zur Darmreinigung, **dadurch gekennzeichnet, dass** die Maisstärke als Körnchen in einer Menge und Form vorliegt, deren Aufnahme einen Blutzuckerspiegel liefert, der dem ähnlich ist, der in gesunden Personen nach einer normalen Mahlzeit oder in einer diabetischen Person, die ihre normale Medikation einnimmt, vorliegt, und keinen Fäzesrückstand hinterlässt.

2. Verwendung nach Anspruch 1, in der der Blutzuckerspiegel während des Verlaufs der Darmreinigung auf mindestens 3,5 mmol/l, vorzugsweise auf etwa 4 mmol/l gehalten wird.

3. Verwendung nach Anspruch 1, wobei es sich bei der Darmreinigung um einen Schritt in der Vorbehandlung von Patienten handelt, für die vorgesehen ist, dass sie einer Koloskopie unterzogen werden.

4. Verwendung nach Anspruch 1, wobei es sich bei der Darmreinigung um einen Schritt in der Vorbehandlung von Patienten handelt, für die vorgesehen ist, dass sie einer Sigmoidoskopie unterzogen werden.

5. Verwendung nach Anspruch 1, wobei es sich bei der Darmreinigung um einen Schritt in der Vorbehandlung von Patienten handelt, für die vorgesehen ist, dass sie einer Bariumeinlauf-Untersuchung der Eingeweide unterzogen werden.

6. Verwendung nach Anspruch 1, wobei es sich bei der Darmreinigung um einen Schritt in der Vorbehandlung von Patienten handelt, für die vorgesehen ist, dass sie einer Darmröntgenuntersuchung unterzogen werden.

7. Verwendung nach Anspruch 1, wobei es sich bei der Darmreinigung um einen Schritt in der Vorbehandlung von Patienten handelt, für die vorgesehen ist, dass sie einer Darmchirurgie unterzogen werden.

8. Verwendung nach Anspruch 1, wobei die Zusammensetzung weiterhin ein einfaches Kohlenhydrat in einem Verhältnis von mindestens 1:5, vorzugsweise mindestens 1:4 und am meisten bevorzugt etwa 1:3 zu dem komplexen Kohlenhydrat umfasst.

9. Verwendung nach Anspruch 8, wobei es sich bei dem einfachen Kohlenhydrat um Saccharose handelt.

10. Zusammensetzung zur oralen Aufnahme, wobei die Zusammensetzung keinen Fäzesrückstand hinterlässt, **dadurch gekennzeichnet, dass** die Zusammensetzung
- native Maisstärke in einer Form, die für eine solche Aufnahme geeignet ist, wobei die Maisstärke als aggregierte Körnchen, die unter Verwendung einer Substanz hergestellt wurden, die aus Gummiarabikum, Kaliumalginat, Guargummi, Methylcellulose, Ethylcellulose, Paraffin, gehärtetem Baumwollsaatöl, Bienenwachs, Carnaubawachs und vorgelatinierter Stärke ausgewählt ist, in einer Menge und Form vorliegt, deren Aufnahme einen Blutzuckerspiegel von mindestens 3,5 mmol/l, vorzugsweise etwa 4 mmol/l liefert, wenn sie in Zeitabständen eingenommen wird, die einem normalen gewöhnlichen Mahlzeitenmuster entsprechen, und
- einfache Kohlenhydrate umfasst, deren Aufnahme einen Blutzuckerspiegel liefert, der dem Blutzuckerspiegel entspricht, der nach Aufnahme einer normalen Mahlzeit erhalten wird.

11. Zusammensetzung nach Anspruch 10, wobei es sich bei dem einfachen Kohlenhydrat um Saccharose handelt.

12. Zusammensetzung nach Anspruch 10, wobei das einfache Kohlenhydrat oder die einfachen Kohlenhydrate und die native Maisstärke in einem Verhältnis von mindestens 1:5, vorzugsweise mindestens 1:4 und am meisten bevorzugt etwa 1:3 vorliegen.

13. Zusammensetzung nach einem der Ansprüche 10 - 12, wobei die native Maisstärke mit einer Schicht aus vorgelatinierter Stärke überzogen ist.

14. Zusammensetzung nach einem der Ansprüche 10 - 12, wobei die Zusammensetzung in der Form eines Pulvers zum Mischen in einer wässrigen oder nicht wässrigen Flüssigkeit ist.

15. Zusammensetzung nach einem der Ansprüche 10 - 12, wobei die Zusammensetzung in der Form einer Brausetablette ist, die zum Lösen in einer wässrigen oder nicht wässrigen Flüssigkeit geeignet ist.

16. Zusammensetzung nach einem der Ansprüche 10 - 12, wobei die Zusammensetzung in der Form einer Tablette oder Kapsel ist, die zur oralen Aufnahme geeignet ist.

## Revendications

1. Utilisation d'amidon de maïs natif pour la fabrication d'une composition destinée à une co-administration lors de procédures de nettoyage intestinal, **caractérisée en ce que** ledit amidon de maïs est présent sous forme de granules en une quantité et sous une forme dont l'ingestion fournit un niveau de glycémie semblable à celui obtenu à la suite d'un repas normal chez des personnes en bonne santé, ou chez une personne diabétique suivant son traitement médicamenteux normalement, et ne laisse pas de résidu fécal.

2. Utilisation selon la revendication 1, dans laquelle le niveau de glycémie est maintenu au moins à 3,5 mmol/l, de préférence à environ 4 mmol/l, pendant ledit nettoyage intestinal.

3. Utilisation selon la revendication 1, dans laquelle le nettoyage intestinal est une étape dans le prétraitement de patients devant subir une coloscopie.

4. Utilisation selon la revendication 1, dans laquelle le nettoyage intestinal est une étape dans le prétraitement de patients devant subir une sigmoïdoscopie.

5. Utilisation selon la revendication 1, dans laquelle le nettoyage intestinal est une étape dans le prétraitement de patients devant subir une exploration des intestins par lavement baryté.

6. Utilisation selon la revendication 1, dans laquelle le nettoyage intestinal est une étape dans le prétraitement de patients devant subir une radiographie intestinale.

7. Utilisation selon la revendication 1, dans laquelle le nettoyage intestinal est une étape dans le prétraitement de patients devant subir une intervention chirurgicale intestinale.

8. Utilisation selon la revendication 1, dans laquelle ladite composition comprend en outre un carbohydrate simple en proportion d'au moins 1:5, de préférence d'au moins 1:4 et de manière davantage préférée d'environ 1:3 par rapport au carbohydrate complexe.

9. Utilisation selon la revendication 8, dans laquelle ledit carbohydrate simple est le sucrose.

10. Composition destinée à l'administration par voie orale, ladite composition ne laissant sensiblement aucun résidu fécal, **caractérisée en ce que** ladite composition contient
- de l'amidon de maïs natif sous une forme appropriée pour une telle ingestion, ledit amidon de maïs étant présent sous forme de granules agrégées, produites en utilisant une substance choisie parmi la gomme arabique, l'alginate de potassium, la gomme guar, la méthyle cellulose, l'éthyle cellulose, la paraffine, l'huile de coton hydrogénée, la cire d'abeille, la cire de carnauba et l'amidon pré-gélatinisé, en une quantité et sous une forme dont l'ingestion fournit un niveau de glycémie d'au moins 3,5 mmol/l, de préférence d'environ 4 mmol/l, lors d'une administration à des intervalles correspondant à un schéma habituel normal de repas, et
- des carbohydrates simples dont l'ingestion fournit un niveau de glycémie correspondant au niveau de glycémie obtenu après l'ingestion d'un repas normal.

11. Composition selon la revendication 10, dans laquelle ledit carbohydrate simple est le sucrose.

12. Composition selon la revendication 10, dans laquelle le ou les carbohydrates simples et l'amidon de maïs natif sont présents en une proportion d'au moins 1:5, de préférence d'au moins 1:4 et de manière davantage préférée d'environ 1:3.

13. Composition selon l'une quelconque des revendications 10 à 12, dans laquelle ledit amidon de maïs natif est enduit d'une couche d'amidon pré-gélatinisé.

14. Composition selon l'une quelconque des revendications 10 à 12, dans laquelle ladite composition se présente sous forme de poudre destinée à être mélangée dans un liquide aqueux ou non aqueux.

15. Composition selon l'une quelconque des revendications 10 à 12, dans laquelle ladite composition se présente sous forme de comprimé effervescent convenant à une dissolution dans un liquide aqueux ou non aqueux.

16. Composition selon l'une quelconque des revendications 10 à 12, dans laquelle ladite composition se présente sous forme de comprimé ou d'une gélule convenant à une ingestion orale.
